# EUROPEAN PATENT SPECIFICATION

(11) **EP 1 403 943 B1**
(45) Date of publication and mention of the grant of the patent: **03.01.2007**
(21) Application number: 03256008.8
(22) Date of filing: 24.09.2003
(51) Int. Cl.: H01M 2/26, H01M 10/48

(54) **Current collector matrix identifier**
Stromabnehmer mit Identifizierung-Matrix
Conducteur de courant avec une matrice identificatrice

(30) Priority: 24.09.2002 US 413076 P
(43) Date of publication of application: 31.03.2004
(73) Proprietor: Wilson Greatbatch Technologies, Inc., Clarence, New York 14031-2033 (US)
(72) Inventor: Miller, Robert, Lancaster, New York 14086 (US)
(74) Representative: Colmer, Stephen Gary

(56) References cited:
- EP-A- 1 204 187
- EP-A- 1 240 995
- US-B1- 6 181 102

## Description

### BACKGROUND OF THE INVENTION.

The present invention relates to the conversion of chemical energy to electrical energy. More particularly, the present invention is directed to the precise regulation of the gram amount of electrode active materials contacted to the opposite sides of a current collector. The precise weight of the current collector is also regulated within strict tolerance. Current collectors that are outside the weight criteria, whether before being contacted with the electrode active material or after, are rejected as being out of tolerance. The strict regulation of the weight of the electrode active material in a cell is particularly important when different active materials are contacted to opposite sides of the current collector. Such a configuration is exemplified by: silver vanadium oxide (SVO)/current collector/fluorinated carbon (CFₓ), and it is important that the weight ratio of active materials is closely regulated for proper cell functioning.

### SUMMARY OF THE INVENTION

The present invention relates to a cell including a cathode having a second cathode active material of a relatively high energy density but a relatively low rate capability sandwiched between two current collectors and with a first cathode active material having a relatively low energy density but a relatively high rate capability in contact with the opposite sides of the current collectors. It is important for proper cell functioning that the weight ratio of the first and second cathode active materials is within a strict tolerance. Further, it is important to be able to track and record this information, as well as other data, for each cell built in a production facility. Marking the current collectors with an identifying I.D. matrix that is read and recorded for each electrode and each cell does this.

The present cell is useful for powering an implantable medical device, such as an automatic implantable cardioverter defibrillator, cardiac pacemaker, neurostimulator, drug pump, bone growth stimulator, and hearing assist device.

In accordance with a further aspect of this invention there is provided an electrical energy storage device which comprises:
a) a first electrode comprising an electrode active material contacted to a support portion of a current collector, wherein the current collector has an exposed tab provided with unique identifying marks;
b) a second, counter electrode;
c) a separator disposed between the first and second electrodes to prevent direct contact between them when they are in electrical association with each other;
d) a casing housing the first and second electrodes; and
e) a first terminal connected to the current collector of the first electrode and a second, opposite polarity terminal connected to the second electrode.

These and other objects of the present invention will become increasingly more apparent to those skilled in the art by reference to the following description and to the appended drawings.

### BRIEF DESCRIPTION OF THE DRAWINGS

Fig. 1 is a perspective view, partly broken away, of an electrochemical cell 10 according to the present invention.
Fig. 2 is a plan view of a current collector 30 including an ID matrix identifier 62.
Fig. 3 is an enlarged view of the indicated area on Fig. 2.
Fig. 4 is an exploded view of one embodiment of a sandwich cathode 32 of the present invention.
Fig. 5 is a flow chart depicting the steps for building a cathode electrode according to the present invention.
Fig. 6 is a flow chart depicting the steps for building an electrochemical cell including the cathode assembled according to Fig. 5.

### DETAILED DESCRIPTION OF THE PREFERRED EMBODIMENTS

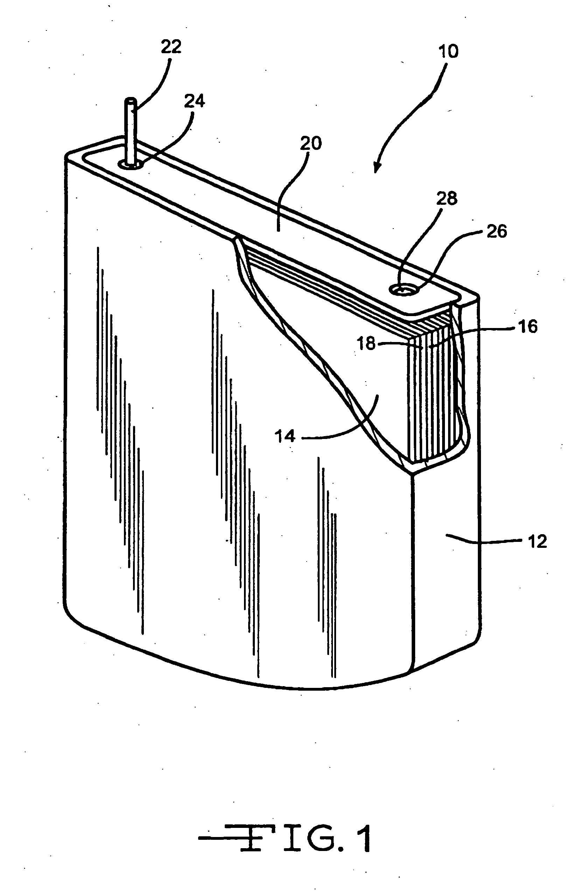

Fig. 1 is a perspective view of an exemplary electrochemical cell 10. The cell 10 includes a casing 12 housing an electrode assembly of an anode electrode comprising a plurality of anode plates 14 and a cathode electrode comprising a plurality of cathode plates 16 prevented from directly contacting each other by an intermediate separator 18. The anode/cathode electrode assembly is in a prismatic configuration housed in the deep-drawn casing 12 closed by a lid 20.

The lid 20 includes an opening supporting a terminal lead 22 insulated from the lid by an insulating glass 24. This structure is commonly referred to as a glass-to-metal seal. The terminal lead 22 is connected to one of the electrodes, typically the current collector (not shown in Fig. 1) for the cathode electrode, and serves as the positive terminal. The current collector for the anode electrode is connected to the casing 12 or lid 20, or both, which serve as the negative terminal. This type of cell construction is referred to as a case-negative configuration. A case-positive configuration has the cathode connected to the case and the negative electrode connected to the terminal lead 22. An activating electrolyte is filled into the other lid opening 26 and a closure member 28 hermetically sealed therein completes the cell 10.

While the exemplary cell 10 shown in Fig. 1 is of a prismatic design, the present invention is not intended to be so limited. In a broader sense, the present system is useful with many different types of cell designs including those of jellyroll or spirally-wound electrode assemblies, button-type cells, coin-cells, and the like. The present system is also useful with capacitors of either an electrochemical, electrolyte or hybrid design. This is what is meant by the term "electrical energy storage device" as used in this description.

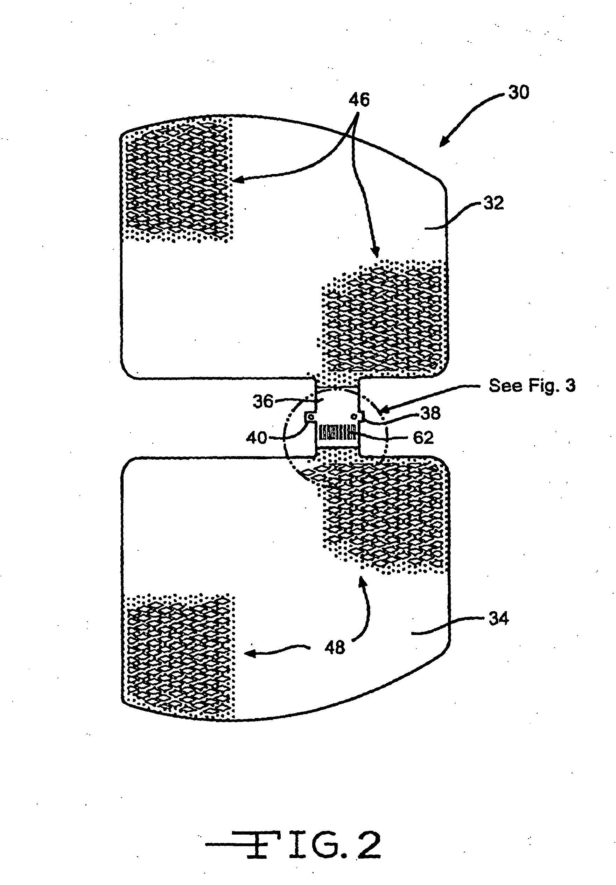

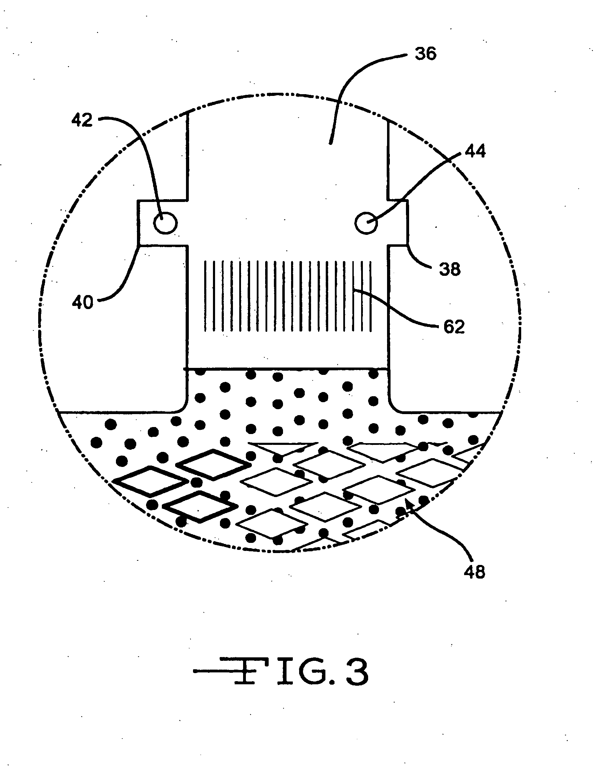

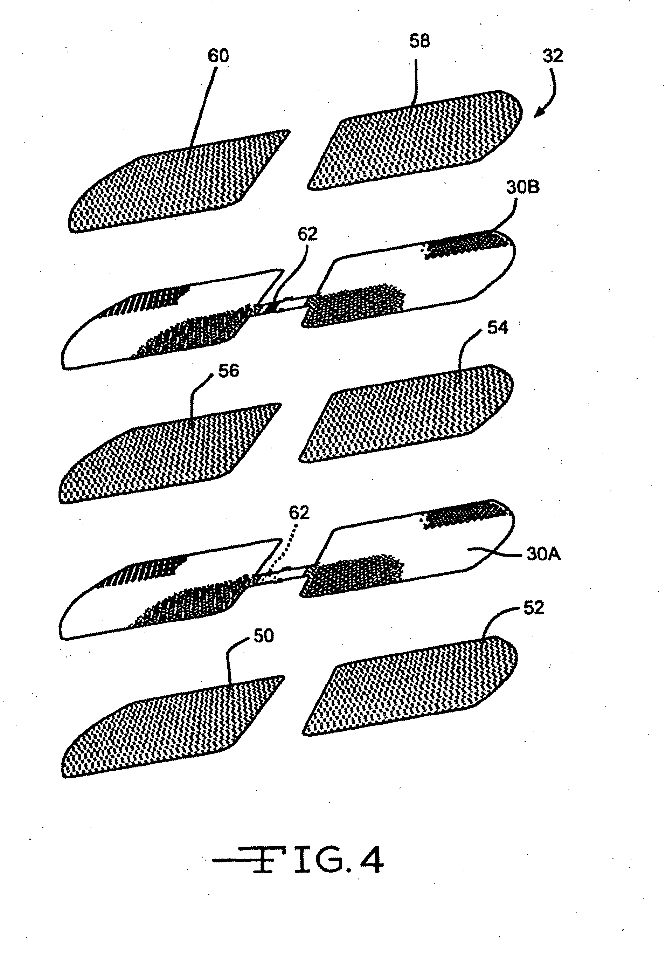

Fig. 2 shows a current collector 30 of a structure useful with the electrode 32 shown in Fig. 4. The illustrated electrode 32 is a cathode, although the present invention is equally applicable to an anode electrode. The cathode comprises a first current collector 30A and a second current collector 30B. The current collectors 30A and 30B are essentially identical and their structure will be described in detail with respect to the illustrated current collector 30 of Figs. 2 and 3.

The current collector 30 comprises opposed wing sections 32 and 34 connected together by an intermediate tab portion 36. The tab 36 supports spaced apart projections 38 and 40. The latter projection 40 has an aperture 42 while an aperture 44 is spaced a short distance inboard from the former one (Fig. 3). The projections 38, 40 and apertures 42, 44 serve as indexing structures for accurately and repeatably positioning the current collector in a fixture for building the electrode, as will be explained in detail hereinafter. The current collector wing sections 32, 34 each comprise an open grid structure 46, 48, respectively, providing them in the form of a screen, and the like. One preferred method for providing the open grid current collectors is described in U.S. Patent No. 6,110,622 and 6,461,771, both to Frysz et al.

As shown in Fig. 4, an electrode, for example a cathode electrode, is built by positioning in an appropriately shaped fixture (not shown) a pair of blanks 50 and 52 of a first electrode active material, for example SVO, followed by the first current collector 30A having its respective wings positioned on top of the blanks. Blanks 54 and 56 of a second electrode active material, for example CFₓ, are positioned on top of the opposite sides of the wings of current collector 30A.

The second current collector 30B is then positioned on top of the second electrode active material blanks 54, 56 opposite the first current collector 30A. Finally, two blanks 58 and 60 of a third electrode active material, for example SVO, are positioned on the wings of the current collector 30B opposite the second electrode active material.

This assembly is then subjected to sufficient pressure to intimately contact the active materials to the opposite sides of the respective current collectors 30A, 30B. Direct bonding contact with the current collector sides is important to prevent delamination. However, it is also important that the SVO and CFₓ materials are segregated to their respective current collector sides so that the active material/current collector interfaces are not "contaminated" by the opposite active material. In other words, it is important that one active material does not migrate through the screen grid to the other side of the current collector to interfere with direct bonding of the other active material to the current collector surface.

The thusly assembly electrode assembly is.referred to as a "sandwich electrode". A preferred form is a cathode electrode with the first and third active materials of a greater rate capability, but a lesser energy density than the intermediate second active material. The second active material has a greater energy density, but a lesser rate capability than the first and third active materials. Silver vanadium oxide is preferred for the first and third active materials while CFₓ is preferred for the intermediate second active material.

In a broader sense, it is contemplated by the scope of the present invention that the first and third active materials of the present sandwich cathode design are any materials that have a relatively lower energy density but a relatively higher rate capability than the second active material. In addition to silver vanadium oxide, copper silver vanadium oxide, V₂O₅, MnO₂, LiCoO₂, LiNiO₂, LiMn₂O₄, TiS₂, Cu₂S, FeS, FeS₂, copper oxide, copper vanadium oxide, and mixtures thereof are useful as the first and third active materials, and in addition to fluorinated carbon, Ag₂O, Ag₂O₂, CuF₂, Ag₂CrO₄, MnO₂ are useful as the second active material. Even SVO is useful as the second active material when copper silver vanadium oxide is the first and third active material. For a more detail description of a "sandwich" electrode design, reference is made to U.S Patent No. 6,551,747.

In order to regulate the manufacturing process for the sandwich electrode, each of the current collectors 30A, 30B is provided with a unique identification code or ID matrix 62. The ID matrix 62 is preferably etched, such as by a laser, onto the connecting tab 36. This provides the matrix with a smaller footprint than a typical bar code, thus minimizing warping of the current collector due to excessive heat. Etching is also preferred because it is permanent and will not contaminate the cell as an ink jet marking system might.

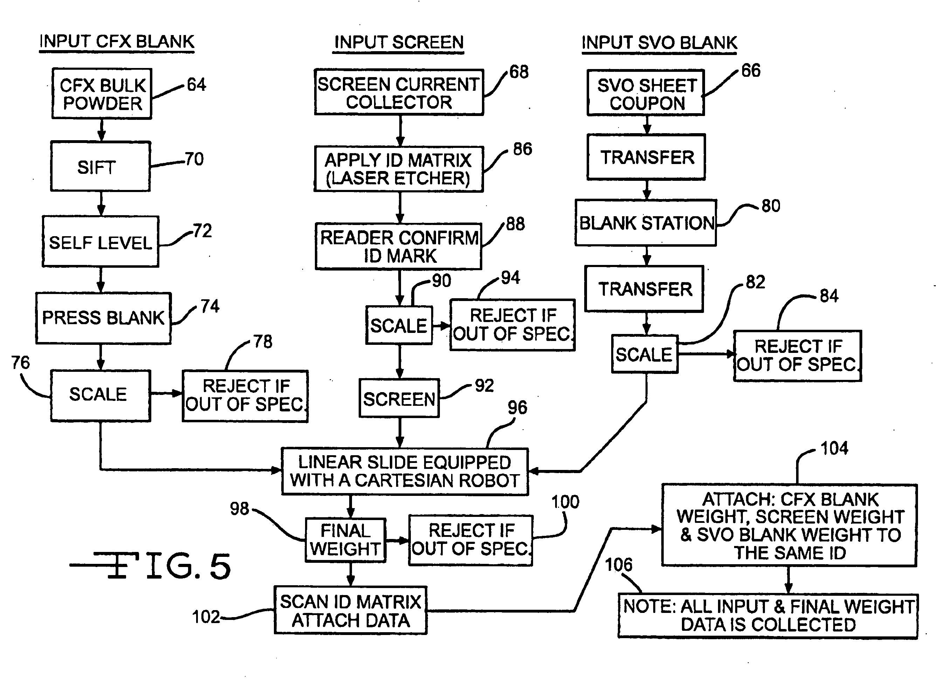

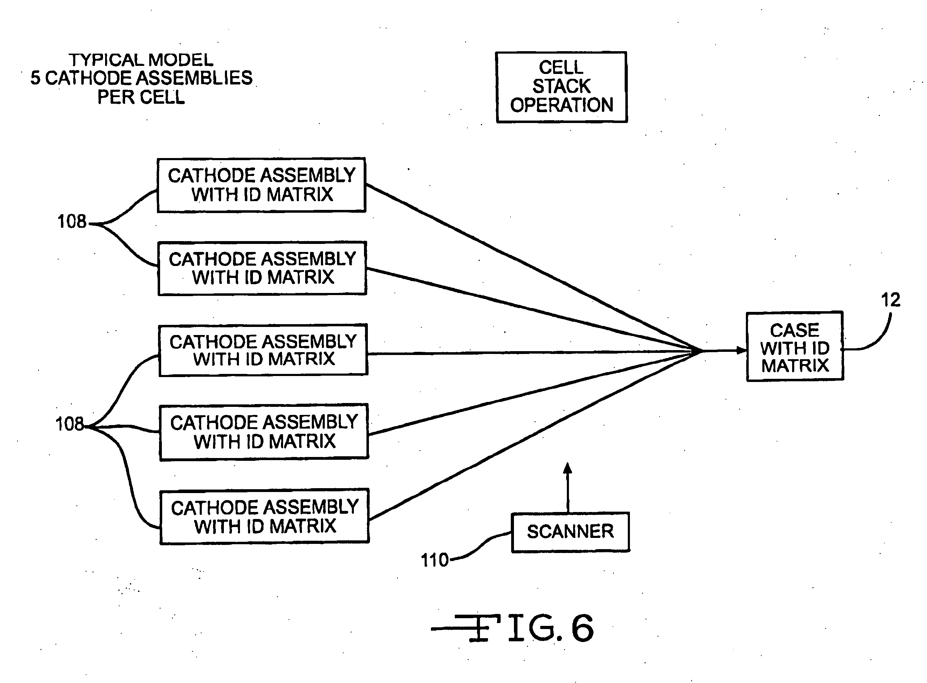

Figs. 5 and 6 are flow charts illustrating an industrial production line for precisely and accurately controlling the processes that constitute the manufacture of the sandwich electrode and, more generally, the associated electrochemical cell 10. The processes begin with a bulk CFₓ powder input 64, a bulk SVO sheet coupon input 66 and a current collector input 68. First moving along the CFₓ flow path, the bulk powder is moved to a sifter 70 that separates out or sieves out any particles greater than a specified size. The sifted out particles are moved to a pulverizer (not shown) that comminutes them to the desired size before they are re-introduced into the sifter. The CFₓ powder leaving the sifter is filled into a fixture having the precise shape of the product cathode electrode. A specified weight amount of CFₓ powder in the fixture is leveled smooth 72 and then pressed with sufficient force to form a blank 74. The blank 74 is weighed on a tare scale 76, and if it is within tolerance, moved to a holding bin. If not, the blank is rejected as being out of specification 78. In order to pass tolerance, a CFₓ blank must be within at least about ±0.1 grams of a specified weight and, more preferably, within about ±0.005 grams of a specified weight.

Formation of an SVO blank takes place in a somewhat different manner. Silver vanadium oxide blank formation is carried out according to the process described in U.S. Patent Nos. 5,435,874 and 5,571,604. As described in the said patents, a freestanding active sheet or coupon is made from SVO of a specified granular size, carbon black or graphite as a conductive additive and a powder fluoro-resin binder such as PTFE powder. These ingredients are mixed in a solvent of either water or an inert organic medium such as mineral spirits. The resulting paste is either run through a series of compacting roll mills to form a thin sheet having a tape form, or it is turned into briquettes that are then calendered into the freestanding sheet as a continuous tape. In any event, the tape is subjected to a drying step that removes any residual solvent or water and then moved to a machine that punches coupons 66 from the tape. The coupons 66 are transferred to a blanking station where a hydraulic press having platens or fixtures presses them into blanks 80 of the precise shape of the product cathode electrode. Each blank 80 is weighed on a tare scale 82, and if it is within tolerance, moved to a holding bin. If not, the blank is rejected as being out of specification 84. In order to pass tolerance, a SVO blank must be within at least about ±0.1 grams of a specified weight and, more preferably, within about ±0.005 grams of a specified weight.

The current collector input 68 begins with a bin holding a plurality of the current collectors 30 (Fig. 2). A chemical machining process, such as described in U.S. Patent Nos. 6,110,622 and 6,461,771.

The current collectors 30 are moved to an etching station 86 where the ID matrix 62 is applied to the connecting tab 36. The etched current collector is moved to a reader 88 that electronically confirms the ID matrix marking 62. After ID matrix confirmation, the current collector is weighed on a tare scale 90, and if it is within tolerance, moved to a holding bin for the etched and weighed current collector screens 92. If not, the current collector is rejected as being out of specification 94. In order to pass tolerance, a current collector must be within at least about ±0.1 grams of a specified weight and, more preferably, within about ±0.006 grams of a specified weight.

The thusly-manufactured CFₓ blank 74, SVO blank 80, and current collectors 92 are then fed to a linear slide equipped with a Cartesian robot 96. This machine is programmable to assemble the three input components into any one of a number of different electrode configurations.

One is of a sandwich cathode as shown in Fig. 4 having the dual wing current collectors 30A, 30B each of a configuration: SVO/current collector/CFₓ/current collector/SVO. Another preferred embodiment is of the same configuration but without the current collectors being of a dual wing construction. Another preferred embodiment is of the configuration: SVO/current collector/SVO/CFₓSVO/current collector/SVO. Still another preferred embodiment is of the configuration: SVO/current collector/CFₓ with the SVO side facing the lithium anode. This latter cathode configuration provides a cell referred to as a "medium-rate design". The others are referred to as being of "high-rate designs".

Regardless of the specific type of cell being built, the finished cathode leaving the Cartesian robot 96 moves to a tare scale 98 where a final weight is recorded. This weight must be within ±5% of the cumulative weights of the respective CFₓ blanks, SVO blanks and current collectors, or the cathode is rejected 100 as being out of specification. After final weighing, the cathode electrode weight is checked and the ID matrix 62 etched onto the current collectors are scanned 102. The ID matrix associated with the readings of the final weights of the various component blanks and current collectors 104 is recorded 106 in the memory of a central processor unit, or it is recorded in some other tangible medium such as on a disk, print out, and the like.

Fig. 6 is a schematic representation of a cell constructed having one or more of the cathode configurations described with respect to Fig. 5. While not shown in the drawing, the cell has an anode as a continuous elongated element or structure of an alkali metal, preferably lithium or a lithium alloy, enclosed within a separator and folded into a serpentine shape. A plurality of cathode electrode assemblies with an associated ID matrix 108 produced according to the component flow chart of Fig. 5 are then interposed between the anode folds. In the case of the cathode shown in Fig. 4, the spaced apart plates are folded relative to the connecting tab 36 so that there is a portion of the anode disposed along opposite major sides or each cathode plate. The cell illustrated in Fig. 6 has two dual wing cathode electrode structures and a fifth cathode plate not of a dual wing construction.

The cathode plates interleaved between the folds of the serpentine anode are fitted inside a suitably sized casing 12 that itself has been provided with a laser etched ID matrix. The case ID matrix is scanned 110 and this data is also recorded for later retrieval. That way, there is a permanent record of each cell detailing the specific electrode configurations with the exact weights of the various active blanks and current collectors housed in a specific casing. The cell is activated with an electrolyte such as of LiPF₆ of LiAsF₆ dissolved in a 50:50, by volume, mixture of propylene carbonate and 1,2-dimethoxyethane. For a case-negative cell design, the current collector of the serpentine anode is connected to the case or lid, or both, and the current collector connecting portions 36 are connected to the terminal lead 22. If a case-positive design is desired, the reverse is true.

One exemplary form of the ID matrix 62 includes a cell model number and a unique serial number. An example is the twenty-character sequence 20770000000000000001. The first four numbers designate the cell as a model 2077 cell, and the following 16 characters are the cell's unique serial number.

In a sandwich electrode design, it is important that the weight ratios of the high rate active material, for example SVO, to that of the high-energy active material, for example CFₓ, be within a strict tolerance. In a lithium electrochemical cell, a sandwich cathode having the configuration of: SVO/current collector/ CFₓ /current collector/SVO, provides for the high volumetric capacity CFₓ active material being quantitatively converted into or used as the high power energy of the SVO material. In that respect, it is believed that during high energy pulsing, the SVO material provides all the discharge energy. Above the discharge voltage of the CFₓ electrode material, only SVO electrode material is discharged, providing all of the energy for pulsing as well as for any background load discharging. Under these discharge conditions, the CFₓ active material is polarized with respect to the SVO material discharge voltages. Then, when the lithium cell is discharged to the working voltage of the CFₓ material, both the SVO and CFₓ materials provide the energy for background load discharging. However, only the SVO material provides energy for high rate pulse discharging. After the SVO active material is pulse discharged, the potential of the SVO material tends to drop due to the loss of capacity. When the SVO background voltage drops below the working voltage of the CFₓ material, the SVO material is charged by the CFₓ material to bring the discharge voltage of the sandwich cathode materials to an equal value. Therefore, it is believed that the SVO material acts as a rechargeable electrode while at the same time the CFₓ material acts as a charger or energy reservoir. As a result, both active materials reach end of service life at the same time.

Thus, it is important for the proper functioning of a lithium cell containing a sandwich cathode of, for example the configuration of: SVO/current collector/CFₓ/current collector/SVO, to have the weights of the respective active materials properly regulated within strict tolerances. This is accomplished by the use of the ID matrix etched onto the current collectors and the casing of the present cells. As previously discussed, other sandwich cathode configurations include: SVO/current collector/SVO/CFₓ/SVO/current collector/SVO and SVO/current collector/CFₓ with the SVO facing the lithium anode. In these alternate embodiments it is also important to strictly regulate the weight ratios of the active materials. The ID matrix can also be etched onto the anode current collector for tracking that component as well.

By "unique identifying marks" to meant herein identifying marks unique to the given current collector.

## Claims

1. An electrical energy storage device (10), which comprises:
a) a first electrode (32) comprising an electrode active material contacted to a support portion of a current collector (30), wherein the current collector has an exposed tab (36) provided with unique identifying marks (62);
b) a second, counter electrode (14);
c) a separator (18) disposed between the first and second electrodes to prevent direct contact between them when they are in electrical association with each other;
d) a casing (12) housing the first and second electrodes; and
e) a first terminal (22) connected to the current collector of the first electrode and a second, opposite polarity terminal connected to the second electrode.

2. The electrical energy storage device of claim 1 wherein the unique identifying marks on the current collector tab are an ID matrix.

3. The electrical energy storage device of claim 1 or claim 2 wherein the unique identifying marks are **characterized** as having been provided on the current collector tab by etching.

4. The electrical energy storage device of any one of the preceding claims wherein the unique identifying marks designate at least a cell serial number.

5. The electrical energy storage device of any one of the preceding claims wherein the unique identifying marks relate to the weight of the current collector.

6. The electrical energy storage device of any one of the preceding claims wherein the unique identifying marks relate to a gram amount of the electrode active material contacted to the support portion of the current collector.

7. The electrical energy storage device of any one of the preceding claims wherein the support portion of the current collector comprises opposed first and second major faces and wherein a first electrode active material contacts the first major face and a second electrode active material contacts the second major face.

8. The electrical energy storage device of claim 7 wherein the first and second electrode active materials are each within about ±0.005 grams of a specified weight.

9. The electrical energy storage device of claim 7 or claim 8 wherein the first electrode is a cathode electrode with silver vanadium oxide and fluorinated carbon contacted to the opposed first and second major faces of the current collector to provide the cathode having the configuration: silver vanadium oxide/current collector/fluorinated carbon.

10. The electrical energy storage device of claim 7, 8 or 9 wherein the first electrode is a cathode electrode comprising two current collectors, each having first and second major faces with exposed tabs provided with unique identifying marks and wherein the cathode electrode has the configuration: silver vanadium oxide/current collector/fluorinated carbon/current collector/silver vanadium oxide.

11. The electrical energy storage device of any one of claims 7 to 10 wherein the current collector comprises wing sections connected together by the tab and wherein each wing section has opposed first and second major faces contacted with an electrode active material.

12. The electrical energy storage device of any one of the preceding claims selected from the group consisting of a prismatic electrochemical cell, a jellyroll electrochemical cell, a button-type cell, a coin cell, an electrochemical capacitor, an electrolyte capacitor, and a hybrid capacitor.

13. An implantable medical device powered by an electrochemical cell which is an electrical energy storage device according to any one of the preceding claims.

14. The implantable medical device of claim 13 when dependent from claims 7 and 9 wherein the unique identifying marks relate to the weight of the current collector and to a gram amount of silver vanadium oxide and fluorinated carbon contacted to the opposed first and second major faces of the current collector support portion.

15. The implantable medical device of claim 13 or claim 14 selected from the group consisting of an automatic implantable cardioverter defibrillator, a cardiac pacemaker, neurostimulator, a drug pump, a bone growth stimulator, and a hearing assist device.

16. A method for producing an electrochemical cell, comprising the steps of:
a) producing a current collector having a support portion intended to be contacted by an electrode active material and a tab;
b) producing unique identifying marks on the current collector tab;
c) contacting an electrode active material to the support portion of the current collector while leaving the tab exposed, thereby providing a first electrode; .
d) producing a second, counter electrode;
e) disposing a separator between the first and second electrodes housed inside a casing with the current collector of the first electrode connected to a first terminal and the second electrode connected to a second terminal; and
f) activating the first and second electrodes with an electrolyte filled into the casing.

17. The method of claim 16 wherein current collector or unique identifying marks produced are defined in any one of claims 2 to 12.

18. The method of claim 16 or claim 17 including scanning the unique identifying marks provided on the current collector tab and recording the associated weights for the current collector, the first electrode active material and the second electrode active material.

19. The method of claim 16, 17 or 18 including providing the casing with case identifying marks.

20. The method of any one of claims 16 to 19 including scanning the case identifying marks and recording the associated weights for the current collector, the first electrode active material and the second electrode active material housed therein.

21. The method of any one of claims 16 to 20 including providing the first and second active materials being ±0.005 grams of a specified weight and the current collector being within ±0.006 grams of a specified weight.

## Patentansprüche

1. Speichervorrichtung für elektrische Energie (10), mit:
a) einer ersten Elektrode (32), die ein elektrodenaktives Material aufweist, das mit einem Stützabschnitt eines Stromabnehmers (30) in Kontakt steht, wobei der Stromabnehmer ein freiliegendes Bezeichnungsschild (36) aufweist, das mit eindeutigen Identifizierungszeichen (62) versehen ist;
b) einer zweiten, Gegenelektrode (14);
c) einem Separator (18), der sich zwischen der ersten und der zweiten Elektrode befindet, um direkten Kontakt zwischen ihnen zu verhindern, wenn sie miteinander in elektrischer Verbindung stehen;
d) einem Gehäuse (12), in dem die erste und die zweite Elektrode untergebracht sind; und
e) einem ersten Anschluss (22), der mit dem Stromabnehmer der ersten Elektrode verbunden ist, und einem zweiten Anschluss mit entgegengesetzter Polarität, der mit der zweiten Elektrode verbunden ist.

2. Speichervorrichtung für elektrische Energie nach Anspruch 1, wobei die eindeutigen Identifizierungszeichen auf dem Stromabnehmer-Bezeichnungsschild eine ID-Matrix sind.

3. Speichervorrichtung für elektrische Energie nach Anspruch 1 oder 2, wobei die eindeutigen Identifizierungszeichen **dadurch gekennzeichnet sind, dass** sie durch Ätzen auf dem Stromabnehmer-Bezeichnungsschild angebracht worden sind.

4. Speichervorrichtung für elektrische Energie nach einem der vorhergehenden Ansprüche, wobei die eindeutigen Identifizierungszeichen wenigstens eine Zellen-Seriennummer angeben.

5. Speichervorrichtung für elektrische Energie nach einem der vorhergehenden Ansprüche, wobei sich die eindeutigen Identifizierungszeichen auf das Gewicht des Stromabnehmers beziehen.

6. Speichervorrichtung für elektrische Energie nach einem der vorhergehenden Ansprüche, wobei sich die eindeutigen Identifizierungszeichen auf einen Grammbetrag des elektrodenaktiven Materials beziehen, das mit dem Stützabschnitt des Stromabnehmers in Kontakt steht.

7. Speichervorrichtung für elektrische Energie nach einem der vorhergehenden Ansprüche, wobei der Stützabschnitt des Stromabnehmers gegenüberliegende erste und zweite Hauptflächen aufweist, und wobei ein erstes elektrodenaktives Material die erste Hauptfläche und ein zweites elektrodenaktives Material die zweite Hauptfläche kontaktiert.

8. Speichervorrichtung für elektrische Energie nach Anspruch 7, wobei das erste und das zweite elektrodenaktive Material jeweils innerhalb von ca. ± 0,005 Gramm eines spezifizierten Gewichts liegen.

9. Speichervorrichtung für elektrische Energie nach Anspruch 7 oder 8, wobei die erste Elektrode eine Kathodenelektrode mit Silber-Vanadium-Oxid und fluoriertem Kohlenstoff ist, die mit den gegenüberliegenden ersten und zweiten Hauptflächen des Stromabnehmers in Kontakt stehen, um die Kathode mit der folgenden Konfiguration bereitzustellen: Silber-Vanadium-Oxid/Stromabnehmer/fluorierter Kohlenstoff.

10. Speichervorrichtung für elektrische Energie nach Anspruch 7, 8 oder 9, wobei die erste Elektrode eine Kathodenelektrode ist, die zwei Stromabnehmer aufweist, die jeweils eine erste und eine zweite Hauptfläche mit freiliegenden Bezeichnungsschildern aufweisen, die mit eindeutigen Identifizierungszeichen versehen sind, und wobei die Kathodenelektrode die folgende Konfiguration hat: Silber-Vanadium-Oxid/stromabnehmer/fluorierter Kohlenstoff/Stromabnehmer/Silber- . Vanadium-Oxid.

11. Speichervorrichtung für elektrische Energie nach einem der Ansprüche 7 bis 10, wobei der Stromabnehmer Flügelabschnitte aufweist, die durch das Bezeichnungsschild miteinander verbunden sind, und wobei jeder Flügelabschnitt gegenüberliegende erste und zweite Hauptflächen aufweist, die mit einem elektrodenaktiven Material in Kontakt stehen.

12. Speichervorrichtung für elektrische Energie nach einem der vorhergehenden Ansprüche, die ausgewählt ist aus der Gruppe bestehend aus einer prismatischen Batteriezelle, einer "Biskuitrollen" (jellyroll)-Batteriezelle, einer Knopfzelle (button-type cell), einer Knopfzelle (coin cell), eines elektrochemischen Kondensators, eines Elektrolytkondensators und eines Hybridkondensators.

13. Implantierbare medizinische Vorrichtung, die von einer Batteriezelle angetrieben wird, die eine Speichervorrichtung für elektrische Energie nach einem der vorhergehenden Ansprüche ist.

14. Implantierbare medizinische Vorrichtung nach Anspruch 13, wenn er von den Ansprüchen 7 und 9 abhängig ist, wobei sich die eindeutigen Identifizierungszeichen auf das Gewicht des Stromabnehmers und auf einen Grammbetrag von Silber-Vanadium-Oxid und fluoriertem Kohlenstoff beziehen, die mit den gegenüberliegenden ersten und zweiten Hauptflächen des Stromabnehmer-Stützabschnitts in Kontakt stehen.

15. Implantierbare medizinische Vorrichtung nach Anspruch 13 oder 14, die ausgewählt ist aus der Gruppe bestehend aus einem automatischen implantierbaren Kardioverter-Defibrillator, einem Herzschrittmacher, einem Neurostimulator, einer Medikamentenpumpe, einem Knochenwachstumsstimulator und einem Hörgerät.

16. Verfahren zur Herstellung einer Batteriezelle, das die folgenden Schritte umfasst:
a) Herstellen eines Stromabnehmers mit einem Stützabschnitt, der dazu ausgelegt ist, mit einem elektrodenaktiven Material und einem Bezeichnungsschild in Kontakt zu treten;
b) Herstellen von eindeutigen ldentifizierungszeichen auf dem Stromabnehmer-Bezeichnungsschild;
c) Kontaktieren eines elektrodenaktiven Materials mit dem , Stützabschnitt des Stromabnehmers, während das Bezeichnungsschild freiliegend bleibt, wodurch eine erste Elektrode bereitgestellt wird;
d) Herstellen einer zweiten, Gegenelektrode;
e) Anordnen eines Separators zwischen der ersten und der zweiten Elektrode, die sich in einem Gehäuse befinden, wobei der Stromabnehmer der ersten Elektrode mit einem ersten Anschluss und die zweite Elektrode mit einem zweiten Anschluss verbunden sind; und
f) Aktivieren der ersten und zweiten Elektroden mit einem in das Gehäuse gefüllten Elektrolyten.

17. Verfahren nach Anspruch 16, wobei der hergestellte Stromabnehmer und die hergestellten Identifizierungszeichen in einem der Ansprüche 2 bis 12 definiert sind.

18. Verfahren nach Anspruch 16 oder 17, das das Scannen der auf dem Stromabnehmer-Bezeichnungsschild vorgesehenen eindeutigen ldentifizierungszeichen und das Aufzeichnen der zugehörigen Gewichte für den Stromabnehmer, das erste elektrodenaktive Material und das zweite elektrodenaktive Material umfasst.

19. Verfahren nach Anspruch 16, 17 oder 18, das das Versehen des Gehäuses mit Gehäuse-Identiftzierungszeichen umfasst.

20. Verfahren nach einem der Ansprüche 16 bis 19, das das Scannen der Gehäuse-Identifizierungszeichen und das Aufzeichnen der zugehörigen Gewichte für den Stromabnehmer, das erste elektrodenaktive Material und das zweite elektrodenaktive Material, die darin untergebracht sind, umfasst.

21. Verfahren nach einem der Ansprüche 16 bis 20, das umfasst, dass vorgesehen wird, dass das erste und das zweite elektrodenaktive Material innerhalb von ± 0,005 Gramm eines spezifizierten Gewichts und der Stromabnehmer innerhalb von± 0,006 Gramm eines spezifizierten Gewichts liegen.

## Revendications

1. Dispositif de stockage d'énergie électrique (10), qui comprend :
a) une première électrode (32) comprenant un matériau actif d'électrode touchant une partie de support d'un collecteur de courant (30), où le collecteur de courant possède une languette exposée (36) munie de marques d'identification uniques (62) ;
b) une seconde contre-électrode (14) ;
c) un séparateur (18) disposé entre la première et la seconde électrodes afin d'empêcher tout contact direct entre celles-ci lorsqu'elles sont en association électrique l'une avec l'autre;
d) un carter (12) contenant la première et la seconde électrodes ; et
e) une première borne (22) reliée au collecteur de courant de la première électrode et une seconde borne de polarité opposée reliée à la seconde électrode.

2. Dispositif de stockage d'énergie électrique selon la revendication 1, dans lequel les marques d'identification uniques situées sur la languette du collecteur de courant sont une matrice identificatrice.

3. Dispositif de stockage d'énergie électrique selon la revendication 1 ou la revendication 2, dans lequel les marques d'identification uniques sont **caractérisées** comme étant prévues sur la languette du collecteur de courant par gravure.

4. Dispositif de stockage d'énergie électrique selon l'une quelconque des revendications précédentes, dans lequel les marques d'identification uniques désignent au moins un numéro de série de cellule.

5. Dispositif de stockage d'énergie électrique selon l'une quelconque des revendications précédentes, dans lequel les marques d'identification uniques concernent le poids du collecteur de courant.

6. Dispositif de stockage d'énergie électrique selon l'une quelconque des revendications précédentes, dans lequel les marques d'identification uniques concernent une quantité de grammes du matériau actif d'électrode touchant la partie de support du collecteur de courant.

7. Dispositif de stockage d'énergie électrique selon l'une quelconque des revendications précédentes, dans lequel la partie de support du collecteur de courant comprend une première et une seconde faces majeures opposées et dans lequel un premier matériau actif d'électrode touche la première face majeure et un second matériau actif d'électrode touche la seconde face majeure.

8. Dispositif de stockage d'énergie électrique selon la revendication 7, dans lequel le premier et le second matériaux actifs d'électrode font chacun environ ± 0,005 gramme d'un poids spécifié.

9. Dispositif de stockage d'énergie électrique selon la revendication 7 ou la revendication 8, dans lequel la première électrode est une électrode cathodique en oxyde de vanadium à l'argent et en carbone fluoré touchant la première et la seconde faces majeures opposées du collecteur de courant afin de fournir la cathode ayant la configuration: oxyde de vanadium à l'argent/carbone fluoré.

10. Dispositif de stockage d'énergie électrique selon la revendication 7, 8 ou 9, dans lequel la première électrode est une électrode cathodique comprenant deux collecteurs de courant, possédant chacun une première et une seconde faces majeures ayant des languettes exposées munies de marques d'identification uniques, et dans lequel l'électrode cathodique possède la configuration : oxyde de vanadium à l'argent/carbone fluoré/collecteur de courant/oxyde de vanadium à l'argent.

11. Dispositif de stockage d'énergie électrique selon l'une quelconque des revendications 7 à 10, dans lequel le collecteur de courant comprend des sections d'ailettes reliées ensemble par la languette, et dans lequel chaque section d'ailette possède une première et une seconde faces majeures opposées touchant un matériau actif d'électrode.

12. Dispositif de stockage d'énergie électrique selon l'une quelconque des revendications précédentes choisi parmi le groupe consistant en une cellule électrochimique prismatique, une cellule électrochimique à enroulement, une cellule de type bouton, une cellule à monnaie, un condensateur électrochimique, un condensateur à électrolyte, et un condensateur hybride.

13. Dispositif médical implantable alimenté par une cellule électrochimique qui est un dispositif de stockage d'énergie électrique selon l'une quelconque des revendications précédentes.

14. Dispositif médical implantable selon la revendication 13 lorsqu'elle dépend des revendications 7 et 9, dans lequel les marques d'identification uniques concernent le poids du collecteur de courant et une quantité de grammes d'oxyde de vanadium à l'argent et de carbone fluoré touchant la première et la seconde faces majeures opposées de la partie de support du collecteur de courant.

15. Dispositif médical implantable selon la revendication 13 ou la revendication 14 choisi parmi le groupe consistant en un défibrillateur à convertisseur de courant continu implantable automatique, un pacemaker cardiaque, un neurostimulateur, une pompe à médicament, un stimulateur de consolidation osseuse, et un dispositif d'assistance respiratoire.

16. Procédé de production d'une cellule électrochimique, comprenant les étapes consistant à :
a) produire un collecteur de courant possédant une partie de support destinée à être touchée par un matériau actif d'électrode et une languette ;
b) produire des marques d'identification uniques sur la languette du collecteur de courant ;
c) faire toucher un matériau actif d'électrode et la partie de support du collecteur de courant tout en laissant la languette exposée, fournissant ainsi une première électrode;
d) produire une seconde contre-électrode ;
e) disposer un séparateur entre la première et la seconde électrodes placées à l'intérieur d'un carter avec le collecteur de courant de la première électrode relié à une première borne et la seconde électrode reliée à une seconde borne ; et
f) activer la première et la seconde électrodes avec un électrolyte rempli dans le carter.

17. Procédé selon la revendication 16 dans lequel un collecteur de courant ou des marques d'identification uniques produites sont définis dans l'une quelconque des revendications 2 à 12.

18. Procédé selon la revendication 16 ou la revendication 17, comprenant le balayage des marques d'identification uniques prévues sur le collecteur de courant et l'enregistrement des poids associés pour le collecteur de courant, le premier matériau actif d'électrode et le second matériau actif d'électrode.

19. Procédé selon la revendication 16, 17 ou 18 comprenant le fait de fournir au carter des marques d'identification de carter.

20. Procédé selon l'une quelconque des revendications 16 à 19, comprenant le balayage des marques d'identification de carter et l'enregistrement des poids associés pour le collecteur de courant, le premier matériau actif d'électrode et le second matériau actif d'électrode contenus à l'intérieur.

21. Procédé selon l'une quelconque des revendications 16 à 20, comprenant le fait de prévoir le premier et le second matériaux actifs comme faisant ± 0,005 gramme d'un poids spécifié, et le collecteur de courant faisant ± 0,006 gramme d'un poids spécifié.
